# EUROPEAN PATENT APPLICATION

(11) **EP 4 778 493 A1**
(43) Date of publication of application: **22.07.2026**
(21) Application number: 25864616.5
(22) Date of filing: 11.11.2025
(51) Int. Cl.: A61C 8/00

(54) **DATA PROCESSING METHOD AND DEVICE, AND STORAGE MEDIUM**

(30) Priority: 12.11.2024 US 202463719374 P
(71) Applicant: Shining 3D Tech Co., Ltd., Hangzhou, Zhejiang 311258 (CN)
(72) Inventor: BANKS, Alan, Hangzhou, Zhejiang 311258 (CN)
(74) Representative: Dai, Simin
(86) International application number: PCT/CN2025/134200
(87) International publication number: WO 2026/103705

(57) **Abstract**

A data processing method, a device and a storage medium. The method includes: acquiring first scan data by scanning an oral cavity of a patient in a first state, wherein an implant in the patient's oral cavity in the first state is mounted with a first scan body, and the first scan data at least includes scan data of the first scan body; acquiring second scan data by scanning an impression in a preset state, wherein the impression is fabricated based on the oral cavity of the patient in the first state, the impression includes a negative impression corresponding to at least a partial structure of the first scan body, a second scan body is mounted in the impression in the preset state, and a partial shape of the second scan body is adapted to a shape of the negative impression, so that the second scan body can be detachably mounted in the negative impression; and the second scan data at least includes scan data of the second scan body and scan data of the impression; and acquiring target data based on the first scan data and the second scan data, wherein the target data is used for fabricating a dental prosthesis of the patient.

## Description

### TECHNICAL FIELD

Embodiments of the present disclosure relate to the technical field of oral implant, and in particular, to a data processing method, a device and a storage medium.

### BACKGROUND

With the continuous development of oral implant technology, implant restoration has become one of the mainstream solutions for dentition defects and missing. The implant restoration typically involves placing an implant into a patient's oral cavity (maxilla or mandible) and then fixing a pre-fabricated prosthesis (e.g., crown, denture, bridge) onto the implant. In the implant restoration process, precisely acquiring pose information of the implant within the patient's oral cavity and correlating the pose information with morphological data of oral soft tissues (e.g., mucosa, gingiva) is prerequisite for ensuring that the subsequently fabricated prosthesis precisely fits the implant mechanically and adapts to the oral soft tissues.

In related arts, to obtain pose data of the implant, an interim scan body can be attached to the implant within the oral cavity. An intra-oral scanner is then used to directly scan the oral cavity, thereby acquiring the pose information of the implant within the oral cavity, as well as a spatial relationship between the implant and soft tissues in the oral cavity, for example, the relative pose between the implant and the gingiva, for use in subsequent prosthesis fabrication. However, during the scanning process, patient's movements such as swallowing and mouth opening easily cause deformation of the soft tissues in the oral cavity, so that the morphology of the soft tissues in the oral cavity reconstructed based on the scan data is greatly different from the real morphology and is not accurate enough. Additionally, some soft tissue areas (for example, a connection area between the implant neck and the gingival cuff) are located in a blind spot for scanning, resulting in missing data, and the relative pose information of the intra-oral soft tissues and the implant cannot be accurately restored, and the prosthesis fabricated based on the relative pose cannot be well attached to the soft tissues, which affects the aesthetics and service life of the prosthesis.

Therefore, it is necessary to provide a solution that can more accurately determine the pose information between the implant and the intra-oral soft tissues.

### SUMMARY

In view of this, the present disclosure provides a data processing method, a device, and a storage medium.

According to a first aspect of the present disclosure, a data processing method is provided, including:
acquiring first scan data by scanning a patient's oral cavity in a first state, where the first state of the patient's oral cavity indicates that at least one first scan body is mounted on an implant in the patient's oral cavity, and the first scan data at least includes scan data of the first scan body;
acquiring second scan data by scanning an impression in a preset state, where the impression is fabricated based on the patient's oral cavity in the first state, the impression includes a negative impression corresponding to at least a part of the first scan body, the impression in the preset state at least indicates that at least one second scan body is mounted in the impression, a partial shape of the second scan body is adapted to a shape of the negative impression, and the second scan body is detachably mounted in the negative impression, and the second scan data at least includes scan data of the second scan body and scan data of the impression; and
acquiring target data based on the first scan data and the second scan data, where the target data is used to fabricate a dental prosthesis of the patient.

According to a second aspect of the present disclosure, an electronic device is provided, including: one or more processors, a memory, and a computer program stored in the memory and executable by the one or more processors, where the one or more processors, when executing the computer program, are caused to perform the method according the first aspect.

According to a third aspect of present disclosure, a non-transitory computer-readable storage medium is provided, where the computer-readable storage medium stores a computer program, and when the computer program is executed, the method in the first aspect is implemented.

According to the solution provided by the present disclosure, when fabricating the dental prosthesis, the patient's oral cavity can be scanned when at least one first scan body is mounted on the implant in the patient's oral cavity to obtain the scan data of the first scan body; meanwhile, wash impression can be performed for the patient's oral cavity in a state that the first scan body is mounted on the implant in the patient's oral cavity to fabricate the impression, the impression includes a negative impression corresponding to at least a partial structure of the first scan body, and a second scan body can be mounted in the impression, where a partial shape of the second scan body is adapted to a shape of the negative impression so that the second scan body can be detachably mounted in the negative impression. In a state where a second scan body is mounted in the impression, the impression is scanned to obtain scan data of the second scan body and scan data of the impression. Then, the scan data of the first scan body and the scan data of the second scan body may be aligned with the scan data of the impression to obtain target data, and the dental prosthesis of the patient is fabricated by using the target data.

By fabricating the impression of the patient's oral cavity, the impression can fully and accurately replicate the morphology of the intra-oral soft tissues, such as marginal contour of the gingival cuff and the fine undulations of the alveolar ridge. Through precise adaptation between the second scan body and the negative impression of the impression, the "implant coordinates" carried by the first scan body are indirectly transferred to the detached impression. This solves the problem of "disconnection between the intra-oral implant coordinates and the detached impression data", thereby providing a "physical correlation basis" for subsequent data alignment. Through the above solution, high-precision correlation between implant coordinates and morphological data of the oral cavity can be realized, and an accurate data basis is provided for the "aesthetic design" of the prosthesis (such as the natural transition between the neck margin of the prosthesis and the gingiva).

It shall be understood that both the foregoing general description and the following detailed description are examples and illustrative only and do not limit the present disclosure.

### BRIEF DESCRIPTION OF DRAWINGS

To describe the technical solutions in the embodiments of the present disclosure more clearly, the following briefly describes the accompanying drawings that need to be used in description of the embodiments, and it is obvious that the accompanying drawings in the following description are merely some embodiments of the present disclosure, and a person of ordinary skill in the art may further obtain other accompanying drawings based on these accompanying drawings without making creative efforts.
FIG. 1 is a schematic diagram of implanting a prosthesis in a patient's oral cavity according to an embodiment of the present disclosure.
FIG. 2 is a flowchart of a data processing method according to an embodiment of the present disclosure.
FIG. 3 is a schematic structural diagram of a third scan body according to an embodiment of the present disclosure.
FIG. 4 is a schematic structural diagram of a first scan body according to an embodiment of the present disclosure.
FIG. 5 is a schematic structural diagram of a second scan body according to an embodiment of the present disclosure.
FIG. 6 is a schematic diagram of fixing a main scan body to a patient's oral cavity according to an embodiment of the present disclosure.
FIG. 7 is a schematic diagram of fixing a healing collar to a patient's oral cavity according to an embodiment of the present disclosure.
FIG. 8 is a schematic diagram of a denture scan body mounted in a wash impression according to an embodiment of the present disclosure.
FIG. 9 is a schematic diagram of a structure of a denture scan body according to an embodiment of the present disclosure.
FIG. 10 is a schematic diagram of a logical structure of a data processing apparatus according to an embodiment of the present disclosure.

### DETAILED DESCRIPTION

The following clearly and completely describes the technical solutions in the embodiments of the present disclosure with reference to the accompanying drawings in the embodiments of the present disclosure. All other embodiments obtained by a person of ordinary skill in the art based on the embodiments of the present disclosure without making creative efforts shall fall within the protection scope of the present disclosure.

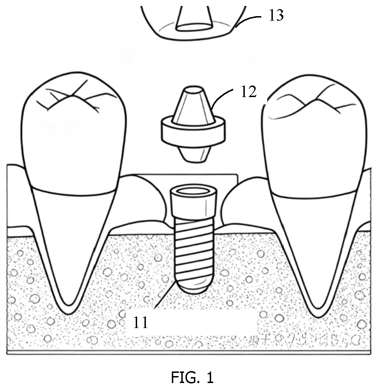

As shown in FIG. 1, implant restoration generally involves placing an implant 11 in a patient's oral cavity (e.g., maxilla or mandible), then mounting an abutment 12 on the implant 11, and fixing a prefabricated prosthesis 13 (e.g., a crown, a bridge, etc.) on the abutment 12.

In the implant restoration process, it is usually necessary to determine a pose relationship between the implant and intra-oral soft tissues, and then the prosthesis is fabricated based on the pose relationship, so that the fabricated prosthesis can be accurately fitted with the implant and perfectly fitted with soft tissues such as gingiva.

In the related art, an interim scan body may be mounted on an implant in the oral cavity, and an intra-oral scene may be directly scanned by an intra-oral scanner, so that pose information of the implant in the oral cavity and a correlation between the implant and intra-oral soft tissues may be obtained. However, the morphology of the intra-oral soft tissues obtained in this manner can be inaccurate, and the relative pose relationship between the intra-oral soft tissues and the implant can also be inaccurate, with the result that the fabricated prosthesis cannot perfectly fit the intra-oral soft tissues.

Taking gingival cuff as an example, which refers to an annular soft tissue area formed around the implant neck after the implant is placed into the alveolar bone of the patient, and a shape, thickness and edge contour of the gingival cuff directly determine the biological closure and aesthetics of the prosthesis. On the one hand, the edge of the prosthesis needs to be accurately attached to the gingival cuff to form a biological barrier around the implant to prevent food residues and bacteria from invading the interface between the implant and the bone to cause inflammation (such as peri-implantitis). On the other hand, the natural radian of the gingival cuff needs to match the shape of the neck margin of the prosthesis, so as to realize the visual coordination of the prosthesis and the natural dentition, and meet the clinical aesthetic needs. Therefore, it is a key link in the digital implanting repair process to accurately obtain the morphological data of the gingival cuff and its relative pose to the implanting, and use the above data to make a prosthesis with a better fit to the intra-oral soft tissues.

In related arts, in order to obtain the pose information of the implant, an interim scan body may be mounted on the implant in the oral cavity, and the intra-oral scene may be directly scanned by an intra-oral scanner, so that the pose information of the implant in the oral cavity and the correlation between the implant and the gingival cuff may be obtained synchronously. However, the gingival cuff belongs to dynamic soft tissues, and during the scanning process, the patient's oral saliva secretion, tongue activity or slight touch of the scanner probe may cause temporary deformation of the gingival cuff (such as soft tissue depression and pulling deformation), so that the shape of the gingival cuff obtained by scanning has a large deviation from the real physiological state. Simultaneously, data loss is prone to occur in the connection area between the implant neck and the gingival cuff (typically only 0.5-1 mm wide) due to scanning blind spots (e.g., when the probe cannot be aligned perpendicularly to the interface), making it impossible to accurately restore the relative positional relationship between the gingival cuff and the implant. Due to the inaccurate shape of the determined gingival cuff, the prosthesis is poorly adapted to the gingival cuff, causing biological complications or aesthetic defects.

Therefore, there is an urgent need for a technical solution that can restore the real morphology of soft tissues such as gingival cuff and accurately associate them with implants.

The data processing method provided in the embodiments of the present disclosure may be performed by various electronic devices such as a scanning device, a mobile phone, a tablet computer, a notebook computer, a physical server, a server cluster, and a cloud server. The scanning device may be a desktop scanner or a handheld scanner, and the scanning device may be an intra-oral scanner or an extra-oral scanner. For example, in some scenarios, a scanning device may scan a patient's oral cavity, a scan body, an impression, and then process obtained scan data. In some scenarios, after a scanning device scans the patient's oral cavity, the scan body, the impression, scan data may be sent to another device (for example, a user mobile phone or a computer) that is in wired or wireless communication with the scanning device, and then the another device processes the scan data.

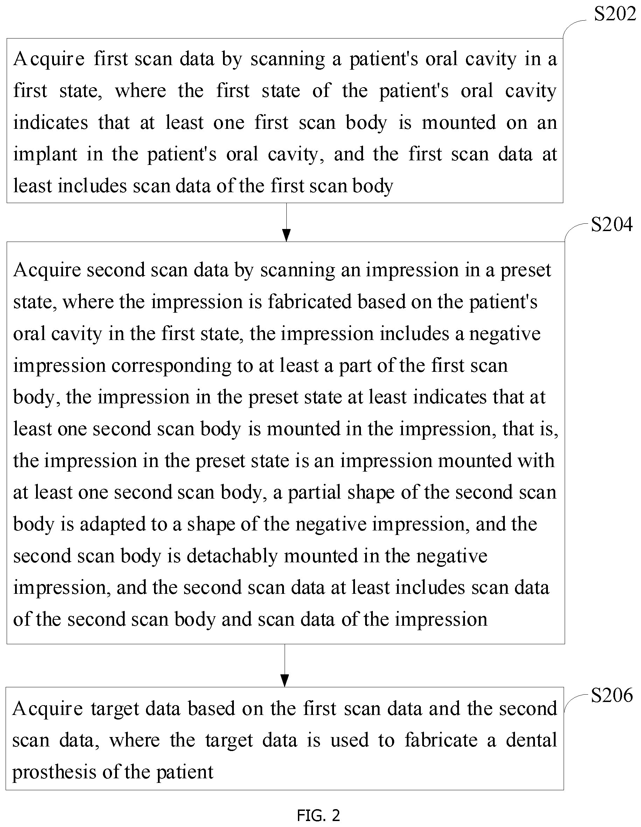

As shown in FIG. 2, the data processing method of the present disclosure may include following steps S202, S204 and S206.

S202, acquiring first scan data by scanning a patient's oral cavity in a first state, where the first state of the patient's oral cavity indicates that at least one first scan body is mounted on an implant in the patient's oral cavity, and the first scan data at least includes scan data of the first scan body.

In step S202, the implant may be placed in the maxilla or mandible of the patient's oral cavity, and then one or more first scan bodies are fixed on the implant directly or indirectly. In some embodiments, the first scan body may be directly fixed on the implant, or in some embodiments, an abutment may be first mounted on the implant, and then the first scan body is fixed on the abutment.

The first scan body is provided with a mounting interface (such as a hexagon socket or a threaded hole) matched with the implant or the abutment, so as to fix the first scan body on the implant or the abutment, and a central axis of the first scan body is collinear with an axis of the implant. As the first scan body is rigidly connected to the implant, the pose of the implant and/or the abutment in the oral cavity may be determined based on the pose (position, angle, etc.) of the first scan body in the patient's oral cavity. In some embodiments, the first scan body may include fiducial markers (for example, one or more of coded fiducial points, non-coded fiducial points, and geometric feature structures, etc.), so as to provide clear positioning features for subsequent scanning, and each fiducial marker can uniquely identify a position feature of one of the first scan bodies.

In addition, the first scan body may include a specific structure, which may form a negative impression in the impression material, and the specific structure can be trapezoidal, hexagonal, quadrangular prismatic, stepped, or stacked rings, etc.

The patient's oral cavity placed with the implant and on which the first scan body is mounted may be referred to as the patient's oral cavity in a first state, and the patient's oral cavity in the first state may be scanned by using the oral cavity scanning device to obtain the first scan data.

The first scan data includes fiducial marker data of the first scan body, when the fiducial marker is a fiducial point, the fiducial marker data in the first scan body may be three-dimensional model data of the fiducial point (a three-dimensional point cloud or a mesh model) or two-dimensional data of the fiducial point. The two-dimensional data of the fiducial point includes a two-dimensional picture in a storage format and/or three-dimensional coordinate information stored in a digital format.

The first scan data includes at least scan data of the first scan body, for example, morphological data of the first scan body (for example, a reconstructed three-dimensional model of the first scan body), and fiducial point data in the first scan body. In some embodiments, the first scan data may also include morphological data of the patient's oral cavity (e.g., a reconstructed three-dimensional model of the patient's oral cavity).

By performing an in-situ scan of the first scan body fixed to the implant, the precise pose of the implant within a global coordinate system of the patient's oral cavity can be directly acquired.

In some embodiments, when scanning the first scan body mounted in the oral cavity to obtain the first scan data, photogrammetry scan may be performed on the first scan body to acquire fiducial marker data (e.g., three-dimensional coordinates of fiducial markers) from the first scan body, where the first scan data includes the fiducial marker data.

In some embodiments, when the first scan body mounted in the mouth is scanned to obtain the first scan data, photogrammetry scan may be performed on the first scan body to obtain the fiducial marker data (such as the three-dimensional coordinates of the fiducial marker) in the first scan body, then optical three-dimensional scanning (such as structured light scanning, laser scanning, etc.) may be performed on the first scan body to obtain the morphological data of the first scan body (or the morphological data of the first scan body and the morphological data of the patient's oral cavity), and then the fiducial marker data in the third scan body and the morphological data are combined, and the first scan data includes the data obtained by combination.

In some embodiments, there are multiple first scan bodies, and each first scan body is provided with multiple continuously distributed fiducial points with encoding information, and each fiducial point can uniquely identify the position feature of one position on the first scan body.

For example, when the first scan data of the patient's oral cavity in the first state is acquired, a first image frame set of the patient's oral cavity in the first state may be acquired, and respective fiducial point data of multiple first scan bodies may be obtained based on the first image frame set; pose information and identification information of each first scan body may be determined based on the fiducial point data of each first scan body, and a standard model corresponding to each first scan body may be retrieved from a standard library based on the identification information; the pose information of multiple first scan bodies may be bound to multiple standard models based on a correspondence between multiple standard models and multiple first scan bodies, respectively, to obtain intermediate standard data; a second image frame set of the patient's oral cavity in the first state may be acquired, and morphological data of the first scan body and morphological data of the patient's oral cavity may be obtained based on the second image frame set; and the intermediate standard data and the morphological data of the patient's oral cavity may be spliced based on a correspondence between each standard model in the intermediate standard data and actual morphological data of the first scan body, to obtain the first scan data.

For example, photogrammetry scan may be performed on the first scan body to obtain the first image frame set, and the fiducial point data of the first scan body may be obtained based on the first image frame set. The fiducial point data of the first scan body includes three-dimensional coordinate information of multiple fiducial points set on a surface of each first scan body and encoding information of multiple fiducial points, identification information of the first scan body may be determined based on the encoding information of multiple fiducial points and the coordinate information of multiple fiducial points (the identification information of the first scan body may be a model of the first scan body), and at this time, the fiducial point data of the first scan body, that is, multiple fiducial points of the first scan body scanned in real time, may be displayed on an interactive interface.

Then, the standard model corresponding to the first scan body may be retrieved from the standard library based on the identification information. The standard library may store truth value information of the fiducial points on the first scan body, for example, store a standard model of the first scan body, where the standard model is a complete, loss-free, error-free reference parameter set of the first scan body in an ideal state, for example, a designed CAD (computer-aided design) model of the first scan body, or a position distribution model of multiple fiducial points in the designed first scan body.

In an embodiment, after the standard model of the first scan body corresponding to the identification information is retrieved, the coordinate system of the standard model is converted into the coordinate system of the fiducial point (such as the camera coordinate system of the oral cavity scanning device), and the positioning information of multiple scanning rods is bound with multiple standard scanning rod models respectively to obtain intermediate standard data, and at this time, the intermediate standard data may be displayed on the interactive interface, that is, the standard models are arranged and displayed according to the position distribution of the first scan bodies in the patient's oral cavity in the camera coordinate system of the oral cavity scanning device.

Then, optical three-dimensional scanning (structured light scanning, laser scanning, etc.) may be performed on the patient's oral cavity in the first state to obtain a second image frame set, the morphological data of the first scan body and the morphological data of the patient's oral cavity are obtained based on the second image frame set, then the standard model converted from the coordinate system may be aligned with the actual morphological data of the first scan body, and the standard model and the morphological data of the patient's oral cavity are fused to obtain the first scan data including both the standard model of the first scan body and the morphological data of the patient's oral cavity.

It should be noted that, in the above process, the standard model retrieved from the standard library may be used as the truth value framework data to adjust the splicing relationship between the first image frame set and the second image frame set in real time, so as to eliminate the accumulated error, and more accurate scan data may be obtained, and the prosthesis designed based on this may be more suitable for the patient's oral cavity. Optionally, the first image frame set is acquired in an illumination light mode, and the second image frame set is acquired in a structured light mode.

S204, acquiring second scan data by scanning an impression in a preset state, where the impression is fabricated based on the patient's oral cavity in the first state, the impression includes a negative impression corresponding to at least a part of the first scan body, the impression in the preset state at least indicates that at least one second scan body is mounted in the impression, that is, the impression in the preset state is an impression mounted with at least one second scan body, a partial shape of the second scan body is adapted to a shape of the negative impression, and the second scan body is detachably mounted in the negative impression, and the second scan data at least includes scan data of the second scan body and scan data of the impression.

In step S204, when the first scan body is fixed on the implant or abutment in the patient's oral cavity, the patient's oral cavity may be subjected to a wash impression treatment to prepare an impression of the patient's oral cavity. For example, an impression material may be coated on an intaglio surface of an impression carrier (for example, a tray), and then the impression carrier may be placed in the patient's oral cavity, and the impression material is lightly pressed for a certain period of time, so that the impression material completely covers the first scan body and the surrounding gingiva, alveolar ridges, etc.; and the impression material is completely solidified, and the impression carrier may be removed to fabricate the impression. The impression includes a negative impression corresponding to at least part of the structure of the first scan body, and a shape of the negative impression is complementary to an outer surface of the first scan body. Moreover, the impression can also accurately reflect the morphology of soft tissues of the patient's oral cavity, such as the morphology of gingival cuffs, alveolar ridges, etc.

The second scan body may be used as a physical hub connecting the first scan body (representing the implant pose) with the impression (intra-oral soft tissue morphology). A partial shape of the second scan body matches a shape of the negative impression, so that the second scan body is detachably mounted in the negative impression. For example, the shape of a portion of the second scan body in contact with the negative impression may be consistent with the outer surface of the first scan body, ensuring that the portion of the second scan body may be embedded into the negative impression of the impression without gaps, and realizing the position alignment of the physical layer.

The impression with at least one second scan body mounted thereon may be defined as an impression in a preset state, and then the impression in the preset state is scanned by using the oral scanning device to obtain the second scan data. The second scan data includes at least scan data of the second scan body, such as morphological data of the second scan body (e.g., a reconstructed three-dimensional model of the second scan body), three-dimensional coordinates of fiducial markers on the second scan body, etc., and scan data of the impression, such as morphological data of the impression (e.g., a three-dimensional model of the impression). The oral cavity scanning device in step S202 and step S204 may be the same to facilitate data merging. In other embodiments, in step S204, a desktop scanning device may also be used to scan the impression in the preset state, so that the second scan data is more accurate.

In some embodiments, there are multiple second scan bodies, and each second scan body is provided with multiple continuously distributed fiducial points with encoding information, and each fiducial point can uniquely identify the position feature of one position on the second scan body. S206, acquiring target data based on the first scan data and the second scan data, where the target data is used to fabricate a dental prosthesis of the patient.

In step S206, the first scan data may be aligned with the second scan data to obtain the target data. Alignment refers to unifying the coordinate system of the first scan data and the second scan data. For example, the first scan body and the implant are coaxial, and the second scan body and the implant are coaxial, so that the first scan data and the second scan data can be aligned based on the above features, that is, the "implant coordinate reference" in the first scan data and the "impression morphological data" in the second scan data are unified through the "scan body feature correlation".

For example, a three-dimensional model of the first scan body may be determined by using the first scan data, and a three-dimensional model of the second scan body may be determined by using the second scan data, and central axes of the two three-dimensional models are collinear, so that the two three-dimensional models may be aligned, and then the first scan data and the second scan data may be aligned to obtain the target data, where the target data may reflect the relative pose relationship between the implant and the oral gingiva data, and may be used to fabricate the dental prosthesis of the patient.

By fabricating the impression of the patient's oral cavity, the impression can fully and accurately replicate the morphology of the intra-oral soft tissues, such as marginal contour of the gingival cuff and the fine undulations of the alveolar ridge. Through precise adaptation between the second scan body and the negative impression of the impression, the "implant coordinates" carried by the first scan body are indirectly transferred to the detached impression. This solves the problem of "disconnection between the intra-oral implant coordinates and the detached impression data," thereby providing a "physical correlation basis" for subsequent data alignment. Through the above solution, high-precision correlation between implant coordinates and morphological data of the oral cavity can be realized, and an accurate data basis is provided for the "aesthetic design" of the prosthesis (such as the natural transition between the neck margin of the prosthesis and the gingiva).

In some embodiments, when the target data is obtained based on the first scan data and the second scan data, first pose information of the implant may be determined based on the scan data of the first scan body, second pose information of the implant may be determined based on the scan data of the second scan body, morphological data of the gingiva in the patient's oral cavity may be determined based on the scan data of the impression, and the first scan data and the second scan data may be aligned based on the first pose information and the second pose information to obtain the target data, where the target data includes the morphological data of the gingiva and the pose information of the implant.

For example, fiducial points may be set on the surface of the first scan body, since the first scan body and the implant are rigidly fixed (axes are collinear and there is no relative displacement), the three-dimensional coordinates (X/Y/Z axis position) and posture parameters (axis inclination angle and rotation direction) of the first scan body in the global coordinate system of the oral cavity are calculated by extracting the fiducial points on the surface of the first scan body, and these data are directly equivalent to the "in-situ real pose" (i.e., the first pose information) of the implant to ensure the accuracy of the pose reference. Then, the second pose information of the implant may be determined based on the scan data of the second scan body, the second scan body is embedded into the negative impression of the impression through morphological adaptation, and the negative impression is a reverse replica of the structure of the first scan body, so there is an accurate transfer relationship between the spatial pose of the second scan body and the first scan body (and the implant), and the "extra-oral transferred pose" (i.e., the second pose information) of the implant may be obtained by extracting the coordinates and poses of the fiducial points of the second scan body, and the second pose information is bound to the impression morphological data. At the same time, the morphological data of the patient's oral gingiva is directionally extracted from the scanned data of the impression, the original morphology of the oral cavity is copied from the impression, and the scanned data can completely restore the three-dimensional contour of the gingiva, including details such as an edge radian of the gingival cuff, the gingival thickness distribution, and the gingival morphology at the alveolar ridge crest, thereby avoiding distortion caused by soft tissue deformation during direct scanning in the oral cavity. Taking the first pose information as a reference (the pose accuracy of in-situ scanning is higher), based on the feature that the axes of both scan bodies are collinear with the axis of the implant, the second pose information is aligned with the first pose information, and the gingival morphological data in the impression scan data is integrated into the unified coordinate system synchronously, and finally the target data including "accurate pose information of the implant + gingival morphological data" is formed, ensuring the spatial alignment of the two types of data.

In some embodiments, the patient has a denture, in addition to that the impression in the preset state indicates that the at least one second scan body is mounted in the impression, the impression is also placed in a patient's denture, and the second scan data further includes morphological data of the denture.

The impression is not only internally provided with a second scan body adapted to fit the negative impression, but also entirely placed inside an existing denture of the patient, and the outer surface of the impression is attached to the intaglio surface of the denture. When the second scan data is acquired, in addition to the original second scan data (for extracting the implant transferred pose) and impression scan data (for extracting the gingival morphology), the complete morphological data of the denture may also be collected, including texture of the denture's tissue surface (an intaglio surface fitted to the alveolar ridge), a cusp-fossa morphology of the bite surface (an outer surface in contact with the opposing teeth), a contour of an adjacent surface (a side surface in contact with an adjacent teeth or an adjacent denture), and an extension range of a denture's edge. Then, the second pose information of the implant in the second scan data may be aligned to the in-situ coordinates based on the first pose information of the implant in the first scan data, and the impression data and the denture morphological data are synchronously driven to be integrated into the unified coordinate system, so that the target data not only includes the implant pose and the gingiva morphology, but also incorporates the morphological features of the denture to form a spatially associated data set of the "implant-gingiva-denture".

By synchronously acquiring the denture morphological data, the target data reflecting the correlation relationship between the implant and the gingiva data of the patient's oral cavity and the correlation relationship between the implant and the existing denture of the patient can be obtained, and then the dental prosthesis that more fits the actual needs of the patient can be designed based on the target data. For example, a bite height of the prosthesis can be adjusted according to the morphology of the bite surface of the denture, and the tissue surface morphology of the prosthesis can be optimized according to a fit degree of the tissue surface of the denture, thereby avoiding bite interference or proximal gap between the new prosthesis and the existing denture of the patient, and improving the coordination of oral function after the restoration.

Considering that in designing the prosthesis, bite data of teeth in a patient's oral cavity is also very critical to the design of the prosthesis. In some embodiments, fourth scan data may also be acquired by scanning the patient's oral cavity in a second state, where the second state of the patient's oral cavity indicates that the patient's denture is mounted in the patient's oral cavity, and the fourth scan data at least includes morphological data of the denture and intra-oral bite data of the denture. Then, the fourth scan data may be associated with the second scan data based on the morphological data of the denture to obtain updated second scan data, and the updated second scan data includes the intra-oral bite data of the denture. The step of acquiring the fourth scan data may be performed before or after step S202.

When the patient mounts his/her own denture (existing denture or temporary prosthetic denture) in the oral cavity, the patient's oral cavity at this time can be defined as the patient's oral cavity in the second state. In this case, the denture fits the alveolar ridge morphology, and forms a natural bite relationship with an opposing teeth (natural teeth or opposing denture), which is consistent with a real oral scene during daily chewing of the patient. Then, an oral cavity scanning device may be used to scan the patient's oral cavity in this state to obtain the fourth scan data, where the fourth scan data includes the morphological data of the denture and the intra-oral bite data of the denture. The morphological data of the denture is homologous to the morphological data of the denture that may be included in the second scan data, such as the fitting texture of the denture tissue surface, the cusp-fossa structure of the bite surface, and the extended contour of the edge. The intra-oral bite data of the denture is static or dynamic parameters related to the bite contact point of the denture and the opposing teeth, the height of the bite gap, the alignment relationship of the cusp-fossa and the bite motion trajectory, and these data cannot be obtained by scanning the detached impression, and need to be collected in the real bite state in the oral cavity. Then, the "morphological data of the denture" may be used as a correlation band, and the denture morphology in the fourth scan data is accurately aligned with the denture morphology in the second scan data (or the reverse morphology of the denture in the impression) through a feature matching algorithm (such as extracting a feature curve of a feature tip point and an edge of a bite surface of the denture), so that the fourth scan data and the second scan data are unified in a coordinate system, and then the "denture intra-oral bite data" in the fourth scan data is integrated into the second scan data to form updated second scan data, and then aligned with the first scan data to obtain the target data. At this time, the second scan data not only retains the original second scan data and impression data, but also incorporates intra-oral bite data reflecting the real bite function of the patient.

The supplementary intra-oral bite data provides an actual reference basis for the bite design of the prosthesis, avoids the problem of "deviation between simulated bite and actual bite" when the bite is designed only based on the detached impression (for example, excessively high bite height designed on the detached model leads to patient masticatory discomfort), ensures that the prosthesis, the existing denture of the patient, and the opposing teeth form a coordinated bite relationship, and improves the chewing function and comfort. In addition, the correlation based on the denture morphological data does not require additional fiducial points on the denture or impression, which simplifies the procedure.

The patient denture may be an existing denture of the patient (an old denture of the patient), or may be an interim denture. The patient's existing denture (the patient's old denture) may be a suction denture, crown, bridge, etc.

In some embodiments, the denture is a temporarily fabricated interim denture, and the interim denture is fabricated based on a following manner: fifth scan data can be acquired by scanning the patient's oral cavity in a third state, the patient's oral cavity in the third state indicates that the patient's oral cavity is in a pre-extraction state (i.e., a pre-surgical state), the fifth scan data at least includes intra-oral bite data of the patient's oral cavity in the pre-extraction state (pre-surgical state), and then an interim denture design model of the patient may be generated based on the fifth scan data, and the interim denture may be fabricated based on the interim denture design model.

The patient's oral cavity prior to surgery may be defined as the patient's oral cavity in the fourth state, the patient's oral cavity before surgery may be the oral cavity where a target tooth position in the oral cavity has not been extracted, and in this state, the bite height, the proximal relationship, and the alveolar ridge morphology of the natural teeth in the oral cavity are all in the natural state that the patient is adapted to for a long time, and are the reference benchmark for the function and morphology adaptation of the interim denture. The oral cavity in this state may be scanned by an intra-oral scanning device to obtain the fifth scan data, where the fifth scan data includes intra-oral bite data (such as a bite contact point, a cusp-fossa alignment relationship, a bite gap size, and left-right side bite balance parameters of a natural dentition) of the patient's oral cavity before extraction. An interim denture design model may then be generated in the dental design software based on the fifth scan data. Based on the design model, an interim denture may be fabricated by using a photocuring 3D printing technology.

As the interim denture replicates the patient's natural bite and oral morphology prior to extraction, it can serve as a "provisional reference" for subsequent prosthesis, and morphology and bite data of the denture can be utilized to assist in prosthesis design.

In some embodiments, when the second scan body mounted on the impression is scanned to obtain the second scan data, the second scan body mounted on the impression may be first subjected to photogrammetry scan to obtain the fiducial marker data (such as three-dimensional coordinates of the fiducial marker) in the second scan body, then the second scan body mounted on the impression may be subjected to optical three-dimensional scanning (such as structured light scanning, laser scanning, etc.) to obtain the second scan body and the morphological data of the impression, and then the fiducial marker data and the morphological data in the second scan body are combined, and the second scan data includes the combined data.

In some embodiments, there are multiple second scan bodies, and each second scan body is provided with multiple continuously distributed fiducial points with encoding information, and each fiducial point can uniquely identify the position feature of one position on the second scan body.

When the second scan data of the impression in the preset state is acquired, a first image frame set of the impression in the second state may be acquired, and respective fiducial point data of multiple second scan bodies may be obtained based on the first image frame set; pose information and identification information of each second scan body may be determined based on the fiducial point data of each second scan body, and a standard model corresponding to each second scan body may be retrieved from a standard library based on the identification information; the pose information of multiple second scan bodies may be bound to multiple standard models based on a correspondence between multiple standard models and multiple second scan bodies, respectively, to obtain intermediate standard data; a second image frame set of the impression in the preset state may be acquired, and morphological data of the second scan body and morphological data of the impression may be obtained based on the second image frame set; and the intermediate standard data and the morphological data of the impression may be spliced based on a correspondence between each standard model in the intermediate standard data and actual morphological data of the second scan body, to obtain the second scan data.

For example, photogrammetry scan may be performed on the second scan body to obtain the first image frame set, and the fiducial point data of the second scan body may be obtained based on the first image frame set. The fiducial point data of the second scan body includes three-dimensional coordinate information of multiple fiducial points set on a surface of each second scan body and encoding information of multiple fiducial points, identification information of the second scan body may be determined based on the encoding information of multiple fiducial points and the coordinate information of multiple fiducial points (the identification information of the second scan body may be a model of the second scan body), and at this time, the fiducial point data of the second scan body, that is, multiple fiducial points of the second scan body scanned in real time, may be displayed on an interactive interface.

Then, the standard model corresponding to the second scan body may be retrieved from the standard library based on the identification information. The standard library may store truth value information of the fiducial points on the second scan body, for example, store a standard model of the second scan body, where the standard model is a complete, loss-free, error-free reference parameter set of the second scan body in an ideal state, for example, a designed CAD (computer-aided design) model of the second scan body, or a position distribution model of multiple fiducial points in the designed second scan body.

In an embodiment, after the standard model of the second scan body corresponding to the identification information is retrieved, the coordinate system of the standard model is converted into the coordinate system of the fiducial point (such as the camera coordinate system of the oral cavity scanning device), and the positioning information of multiple scanning rods is bound with multiple standard scanning rod models respectively to obtain intermediate standard data, and at this time, the intermediate standard data may be displayed on the interactive interface, that is, the standard models corresponding to the second scan bodies are displayed in the interactive interface according to the position distribution of the second scan bodies in the impression in the camera coordinate system of the oral cavity scanning device.

Then, optical three-dimensional scanning (structured light scanning, laser scanning, etc.) may be performed on the second scan body in the preset state to obtain a second image frame set, the morphological data of the second scan body and the morphological data of the impression may be obtained based on the second image frame set, then the standard model after coordinate system conversion may be aligned with the actual morphological data of the second scan body, and the standard model and the morphological data of the impression may be fused to obtain the second scan data including both the standard model of the second scan body and the morphological data of the impression.

It should be noted that, in the above process, the standard model retrieved from the standard library may be used as the truth value framework data to adjust the splicing relationship between the first image frame set and the second image frame set in real time, so as to eliminate the accumulated error, and more accurate scan data may be obtained, and the prosthesis designed based on this may be more suitable for the patient's oral cavity. Optionally, the first image frame set is acquired in an illumination light mode, and the second image frame set is acquired in a structured light mode.

In other embodiments, the standard model corresponding to the second scan body may be a stored standard model of other types of scanning rods, such as the standard model of the first scan body, because the first scan body and the second scan body have a one-to-one correspondence, for example, the first scan body of type "A" is mounted on a first tooth, and a structure of the first scan body of type "A" is adapted to a structure of the second scan body of type "a", the identified second scan body of type "a" can be directly replaced with the first scan body of type "A".

For edentulous or partially edentulous patients, it is usually necessary to place multiple implants in the patient's oral cavity and attach a scan body to each implant to determine the intra-oral pose of each implant as well as the relative poses among the implants. Due to limited scanning viewing angle of the oral cavity scan body, each frame of scan data may only include a part of the scan body, and multiple frames of scan data need to be spliced based on the fiducial markers in the scan body to obtain the complete oral cavity shape and the pose relationship of multiple scan bodies. Considering that the shape of the first scan body is limited due to the need for impression treatment, the first scan body cannot be extended in a large range in the oral cavity, that is, the distribution range of the fiducial markers in the first scan body is limited, resulting in poor accuracy of the splicing result when splicing multiple frames of scan data based on the fiducial markers. In order to solve the above problem, in some embodiments, the first scan body includes multiple fiducial markers, and sixth scan data of the patient's oral cavity in a fourth state may also be acquired, the patient's oral cavity in the fourth state indicates that at least one third scan body is fixed in the implant in the patient's oral cavity, where the third scan body includes multiple fiducial markers, and coverage of multiple fiducial markers in the third scan body in the patient's oral cavity is greater than coverage of multiple fiducial markers in the first scan body in the patient's oral cavity. The sixth scan data includes at least scan data of the third scan body, and based on the scan data of the third scan body, the sixth scan data may be associated with the first scan data to obtain updated first scan data, and then aligned with the second scan data to obtain the target data.

At least one third scan body may be fixed on the implant in the patient's oral cavity, and the patient's oral cavity at this time is defined as the patient's oral cavity in the fourth state. The third scan body includes multiple fiducial markers (for example, fiducial points), and coverage of multiple fiducial markers in the third scan body in the patient's oral cavity is greater than coverage of multiple fiducial markers in the first scan body in the patient's oral cavity. For example, the volume of the first scan body is relatively limited, the fiducial markers in the first scan body may only cover a spatial range around the implant, and the third scan body may be designed to have a laterally extending structure (a paddle portion), so that the fiducial markers on a surface of the third scan body may not only cover an area around the implant, but also extend to a surrounding tooth area. For example, the laterally extending structures (paddle portions) of the third scan body may be arranged along a dental arch, so that the intra-oral scanning device can capture the third scan body on successive frames when continuously photographing in the patient's oral cavity, thereby improving the accuracy of the finally obtained target data. Then, sixth scan data may be acquired by scanning in the patient's oral cavity, and the scan data at least includes scan data of the third scan body, such as morphological data of the third scan body and/or fiducial marker data of the third scan body (such as three-dimensional coordinates and/or encoding information of the fiducial marker). As the third scan body and the implant are coaxial, and the first scan body and the implant are also coaxial, the scan data of the third scan body and the scan data of the first scan body can be aligned based on the above feature, and can be unified into the same coordinate system, at the same time, the position and pose information of the implant in the oral cavity and the relative position and pose information between implants carried in the scan data of the third scan body can be integrated into the original first scan data, to finally obtain updated first scan data. The updated first scan data is aligned with the second scan data to obtain target data, so as to fabricate the prosthesis by using the target data.

As the third scan body has a larger fiducial marker coverage, a more stable space constraint can be provided, and the scan data of the third scan body is integrated into the scan data of the first scan body, which can improve the accuracy of the finally determined pose information of the implant and the relative pose between the implants.

In some embodiments, when the third scan body mounted in the oral cavity is scanned to obtain the sixth scan data, photogrammetry scan may be performed on the third scan body to obtain fiducial marker data (e.g., three-dimensional coordinates of the fiducial marker) in the third scan body, and the fiducial marker data is used as the sixth scan data.

In some embodiments, when the third scan body mounted in the mouth is scanned to obtain the sixth scan data, the third scan body may be first subjected to photogrammetry scan to obtain the fiducial marker data (such as the three-dimensional coordinates of the fiducial marker) in the third scan body, then the third scan body may be subjected to optical three-dimensional scanning (such as structured light scanning, laser scanning, etc.) to obtain the morphological data of the third scan body (or the morphological data of the third scan body and the morphology of the patient's oral cavity), and then the fiducial marker data and the morphological data in the third scan body are combined as the sixth scan data.

In some embodiments, there are multiple third scan bodies, and each third scan body is provided with multiple continuously distributed fiducial points with encoding information, and each fiducial point can uniquely identify the position feature of one position on the third scan body.

For example, when the sixth scan data of the patient's oral cavity in the fourth state (the patient's oral cavity is mounted with the third scan body) is acquired, a first image frame set of the patient's oral cavity in the fourth state may be acquired, and respective fiducial point data of multiple third scan bodies may be obtained based on the first image frame set; pose information and identification information of each third scan body may be determined based on the fiducial point data of each third scan body, and a standard model corresponding to each third scan body may be retrieved from a standard library based on the identification information; the pose information of multiple third scan bodies may be bound to multiple standard models based on a correspondence between multiple standard models and multiple third scan bodies, respectively, to obtain intermediate standard data; a second image frame set of the patient's oral cavity in the fourth state may be acquired, and morphological data of the third scan body and morphological data of the patient's oral cavity may be obtained based on the second image frame set; and the intermediate standard data and the morphological data of the patient's oral cavity may be spliced based on a correspondence between each standard model in the intermediate standard data and actual morphological data of the third scan body, to obtain the sixth scan data.

For example, photogrammetry scan may be performed on the third scan body to obtain the first image frame set, and the fiducial point data of the third scan body may be obtained based on the first image frame set. The fiducial point data of the third scan body includes three-dimensional coordinate information of multiple fiducial points set on a surface of each third scan body and encoding information of multiple fiducial points, identification information of the third scan body may be determined based on the encoding information of multiple fiducial points and the coordinate information of multiple fiducial points (the identification information of the third scan body may be a model of the third scan body), and at this time, the fiducial point data of the third scan body, that is, multiple fiducial points of the third scan body scanned in real time, may be displayed on an interactive interface.

Then, the standard model corresponding to the third scan body may be retrieved from the standard library based on the identification information. The standard library may store truth value information of the fiducial points on the third scan body, for example, store a standard model of the third scan body, where the standard model is a complete, loss-free, error-free reference parameter set of the third scan body in an ideal state, for example, a designed CAD model of the third scan body, or a position distribution model of multiple fiducial points in the designed third scan body.

In an embodiment, after the standard model of the third scan body corresponding to the identification information is retrieved, the coordinate system of the standard model is converted into the coordinate system of the fiducial point (such as the camera coordinate system of the oral cavity scanning device), and the positioning information of multiple scanning rods is bound with multiple standard scanning rod models respectively to obtain intermediate standard data, and at this time, the intermediate standard data may be displayed on the interactive interface, that is, the standard models are arranged and displayed according to the position distribution of the third scan bodies in the patient's oral cavity in the camera coordinate system of the oral cavity scanning device.

Then, optical three-dimensional scanning (structured light scanning, laser scanning, etc.) may be performed on the patient's oral cavity in the fourth state to obtain a second image frame set, the morphological data of the third scan body and the morphological data of the patient's oral cavity are obtained based on the second image frame set, then the standard model converted from the coordinate system may be aligned with the actual morphological data of the third scan body, and the standard model and the morphological data of the patient's oral cavity are fused to obtain the sixth scan data including both the standard model of the third scan body and the morphological data of the patient's oral cavity.

It should be noted that, in the above process, the standard model retrieved from the standard library may be used as the truth value framework data to adjust the splicing relationship between the first image frame set and the second image frame set in real time, so as to eliminate the accumulated error, and more accurate scan data may be obtained, and the prosthesis designed based on this may be more suitable for the patient's oral cavity. Optionally, the first image frame set is acquired in an illumination light mode, and the second image frame set is acquired in a structured light mode.

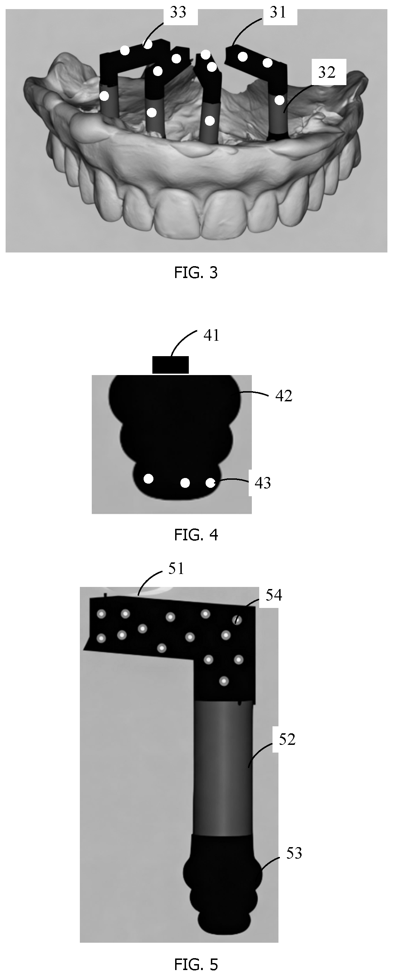

In some embodiments, as shown in FIG. 3, the third scan body includes a paddle portion 31 and a body extension 32, one end of the body extension 32 is configured to be connected to the implant in the patient's oral cavity, and another end is connected to the paddle portion 31, where a surface of the paddle portion and/or the body extension is provided with multiple fiducial markers 33. In FIG. 3, a third scan is located in the impression, and the impression is placed on the denture. For example, the body extension may be a cylindrical or prismatic structure. One end of the body extension is provided with a connection interface that precisely fits the implant or abutment, and the central axis of the body extension can be collinear with the axis of the implant, ensuring the accuracy of implant pose transfer. The other end of the body extension is vertically fixedly connected to the paddle portion to form an L-shaped structure in which the body extension serves as a vertical leg and the paddle portion serves as a horizontal leg. The paddle portion may be a rectangular structure that extends outward horizontally in a direction perpendicular to the axis of the body extension. An extension range of the paddle portion can cover surfaces of adjacent teeth around the implant, the edge of the alveolar ridge, or areas near the oral vestibule (adapted according to the patient's oral dimensions), thereby expanding the coverage area of the fiducial markers within the oral cavity. A surface of the body extension and/or the paddle portion is provided with multiple fiducial markers, such as fiducial points (which may be encoding points or non-encoding points). A relative positional relationship between multiple fiducial markers is known, for example, taking the fiducial marker being the fiducial point as an example, a distance and an angle between fiducial points are known.

In some embodiments, the other end of the body extension 32 is connected to the paddle portion 31, so that the body extension 32 and the paddle portion 31 are in an elongated strip structure.

In some embodiments, the other end of the body extension 32 is connected to the paddle portion 31, so that the body extension 32 and the paddle portion 31 are in an L-shaped structure.

By designing the third scan body as an L-shaped structure and by the vertical layout of the body extension and the paddle portion, the fiducial marker distribution limitation of the traditional scan body in "single axial direction" is broken, and the horizontal extension of the paddle portion enables the fiducial marker to reach the adjacent teeth, alveolar ridges and other areas of the implant, thereby expanding the coverage of the fiducial marker in the oral cavity and providing more spatial position constraints. In the scenario of parallel restoration for multiple implants, the fiducial markers on the paddle portions of adjacent third scan bodies can create spatially overlapping coverage. This enables coordinate linkage among the multiple implants, providing a more comprehensive spatial reference for subsequent prosthesis design and further reducing prosthesis adaptation errors caused by insufficient data dimensions.

For example, the third scan body may retain its core basic structure (such as the L-shaped paddle portion, the body extension, and the fiducial marker mentioned above), and at the same time, a detachable interface (such as a clamping groove with a positioning buckle, an internal threaded hole, or a hexagonal positioning groove) may be provided at the end of the body extension away from the paddle portion for detachably connecting a mating portion. A shape of the mating portion may be designed to match the shape of the first scan body. After the mating portion is connected to the third scan body, the formed overall structure is the second scan body, and the second scan body not only retains the fiducial markers in the paddle portion of the third scan body (for extracting coordinates during subsequent scanning and realizing the correlation with the first scan data), but also has the mating portion consistent with the first scan body structure (for accurately embedding the negative impression of the impression fabricated based on the first scan body to realize the coordinate transfer at the physical level), while meeting the dual functional requirements of "positioning feature retention" and "negative impression adaptation".

For the third scan body, through the modular design of the "basic main body + detachable mating portion", and the mating portion is designed to be adapted to the structure of the first scan body, so that the multiplexing of the scan body modules can be realized, which is more flexible. In some embodiments, after the target data is obtained, a design model of the dental prosthesis of the patient may be generated based on the target data. As the target data includes the pose information (three-dimensional coordinates, axis inclination angle) of the implant, the morphological data of the gingiva (the edge contour of the gingival cuff, and the thickness distribution of the alveolar ridge), the morphology of the denture and the intra-oral bite data (the bite contact point, the cusp-fossa alignment relationship, and the adjacent surface contour), based on the target data, a prosthesis that can be accurately adapted to the implant and can fit the soft tissue of the oral cavity can be designed.

In some embodiments, after the design model of the dental prosthesis of the patient is generated, the design model of the dental prosthesis may also be sent to a fabricating device, and after the fabricating device fabricates the dental prosthesis, a user request for modifying the design model of the dental prosthesis is received in real time, and a revised design model of the dental prosthesis is output according to the user request and the target data. The fabricating device may be a device for fabricating a dental prosthesis, such as a 3D printing device or a milling machine.

The patient's dental prosthesis may be the patient's crown, bridge, or denture, etc.

In some embodiments, as shown in FIG. 4, the first scan body has a first end portion 41 and a second end portion 42, the first end portion 41 is configured to be connected to the implant or the abutment, the second end portion 42 can form the negative impression in the impression material, and the outer surface of the second end portion 42 of the first scan body is provided with multiple fiducial markers 43.

For example, the first end is a connecting end, and is provided with an interface (such as an internal hexagonal positioning groove or a matching thread) adapted to the implant or the abutment, so as to be fixed on the implant or the abutment. After the first scan body is fixed on the implant or the abutment, the first scan body is collinear with the central axis of the implant or the abutment. The second end is a negative impression forming end shaped to form a negative impression in the impression material. The outer surface of the second end portion is provided with multiple fiducial markers (for example, fiducial points), and the relative position relationship of multiple fiducial markers is known and can exist in a standard library for accurate positioning.

In some embodiments, as shown in FIG. 4, the second end portion 42 is shaped as stacked rings, and diameters of the stacked rings gradually decrease in a direction away from the first end portion 41. Such configuration facilitates impression removal. For example, a bottom ring has an outer diameter of 6.0 mm, a middle ring 5.5 mm, and a top ring 5 mm. For example, a bottom ring has an outer diameter of 9 mm, a middle ring 8 mm, and a top ring 7 mm. For example, a bottom ring has an outer diameter of 8 mm, a middle ring 7 mm, and a top ring 6 mm. And so on, which is not limited.

In some embodiments, axes of the stacked rings are collinear.

By designing the second end portion as a structure of stacked rings with "increasing diameter + collinear axis", the impression material can be guided to be fully filled from the bottom ring to the top ring without risk of bubble residue, the shape of the formed negative impression is completely complementary to the second end portion, and "gap-free adaptation" can be realized when the second scan body is embedded subsequently, thereby ensuring the physical basis of coordinate transfer.

In some embodiments, as shown in FIG. 5, the second scan body includes a paddle portion 51, a body extension 52 and a mating portion 53, one end of the body extension 52 is connected to the mating portion 53, another end is connected to the paddle portion 51, a shape of the mating portion 53 is adapted to the shape of the negative impression of the first scan body, so that the mating portion 53 can be mounted in the negative impression of the first scan body, the surface of the paddle portion and/or the body extension is provided with multiple fiducial markers 54, and the relative position relationship between multiple fiducial markers 54 is known.

In some embodiments, the other end of the body extension 52 is connected to the paddle portion 51, so that the body extension 52 and the paddle portion 51 are in an elongated strip structure.

In some embodiments, the other end of the body extension 52 is connected to the paddle portion 51, so that the body extension 52 and the paddle portion 51 are in an L-shaped structure.

In some embodiments, the paddle portion, the body extension, and the mating portion may be integrally formed.

In some embodiments, at least one of the paddle portion or the mating portion is detachably connected to the body extension.

In some embodiments, the mating portion is configured as stacked rings, and a diameter of each of the stacked rings gradually decreases in a direction away from the body extension.

The second scan body may be composed of a paddle portion, a body extension and a mating portion, where the body extension serves as a connecting member, which may be a cylindrical or prismatic solid structure, one end of the body extension is fixedly or detachably connected to the mating portion, and the other end of the body extension is connected to the paddle portion to form an L-shaped structure with "the body extension being a vertical leg and the paddle portion being a horizontal leg". This structure allows the paddle portion to extend to surrounding areas of the implant, thereby expanding coverage of scanning marks (fiducial markers). The mating portion is a key component adapted to the negative impression of the first scan body, and is designed as stacked rings, and the diameter of each ring gradually decreases in the direction away from the body extension, for example, a top ring has an outer diameter of 6.0 mm, a middle ring 5.5 mm, and a bottom ring is 5 mm. In another example, a top ring has an outer diameter of 9 mm, a middle ring 8 mm, and a bottom ring 7 mm. In another example, a top ring has an outer diameter of 8 mm, a middle ring 7 mm, and a bottom ring 6 mm. And so on, which is not limited. The stacked rings are completely complementary to the stacked rings with the "increasing diameter" of the second end portion of the first scan body, so as to ensure that the mating portion can be embedded into the negative impression of the impression without any gap, and realize the position alignment in the physical level.

At the same time, the surface of the paddle portion and/or the body extension is provided with multiple fiducial markers (such as fiducial points), and the relative position relationship between the fiducial markers is pre-calibrated (for example, the spacing and angle parameters are known) to provide a clear feature reference for the scanned data correlation.

In the forming manner, the scan body may be integrally formed, for example, the paddle portion, the body extension, and the mating portion are formed by one-time processing of medical-grade materials, or a partially detachable design may be used, for example, the paddle portion or the mating portion is connected to the body extension through a connection interface, which is convenient to replace mating portions or paddle portions of different specifications according to clinical requirements.

In some embodiments, the impression may be fabricated by: fixing a first scan body on an implant in a patient's oral cavity, uniformly coating an impression material on an intaglio surface of an impression carrier, placing the impression carrier in the patient's oral cavity and covering the first scan body to fabricate an impression including a negative impression of the first scan body.

The impression material may be selected from highly flowable, low-shrinkage medical-grade impression materials (e.g., addition-cure silicone rubber). This material needs to possess sufficient flowability to cover details such as the stacked rings and fiducial markers on the first scan body, while exhibiting minimal volumetric shrinkage after setting to avoid distortion of the negative mold morphology.

In some embodiments, the impression carrier includes any of: an interim denture of the patient, an existing denture of the patient, a custom tray of the patient, a stock tray.

The following describes the data processing method in this embodiment of the present disclosure with reference to a specific embodiment.

The healing collar in this embodiment is the first scan body mentioned above, the denture scan body in this embodiment is the second scan body mentioned above, and the primary scan body in this embodiment is the third scan body mentioned above.

In the art of fabricating and installing oral prosthesis (especially full-arch oral prosthesis), a patient's existing teeth are removed, and implants and abutments are installed. The prosthesis, namely the full-arch prosthesis, is fabricated and then secured in place through the abutments. When fabricating the prosthesis, the location and position of the abutments are critical to the proper seating of the prosthesis within the patient's oral cavity.

Historically, the location and position of the abutments have been determined through physical impressions, and then the prosthesis is fabricated from information obtained from these impressions. Modern technology employs digital imaging to create digital models from which the prosthesis is fabricated. For example, post-surgical digital imaging of the abutments take place directly within the patient's oral cavity.

Common digital imaging techniques include optical three-dimensional scanning (intra-oral scanning, IOS) or photogrammetry (PG). Optical 3D scanning employs a device that emits electromagnetic radiation (e.g. light or laser) that contacts objects within the mouth (e.g. teeth and tissue), and then with the use of image scanners and point clouds, a digital model is produced. In order to determine the location and position of the abutments, devices (often referred to as scan bodies) which have a scannable feature, are temporarily installed into the abutments during the IOS process.

PG employs photographic equipment or a scanning device to collect a plurality of images of the desired object and, using triangulation algorithms to produce a 3D digital model. Those skilled in the art appreciate that PG methodologies provide maximum precision when known dimensions are captured by the photographic images. It is therefore common to temporarily install scan bodies into the abutments, where the scan bodies include fiducial markers, which are often referred to as targets, that have known distances (e.g. the scan bodies include a plurality of dots).

While PG methodologies are useful for producing very accurate 3D digital models of the location and position of the abutments, PG does not provide useful imaging of the teeth and tissue.

In order to fabricate useful prosthesis, information regarding the original bite pattern is extremely useful. A patient's original bite pattern can be captured through physical impressions or using IOS. The limitations of PG imaging typically prevents it's use in pre-surgical imaging of teeth and tissue. The pre-surgical data (i.e. data obtained from physical impressions or IOS) can then be correlated, which is also referred to as stacking, with the post-surgical imaging of the location and position of the abutments.

Additional challenges exist when a patient does not have teeth (edentulous patient) prior to surgery. These patients, who are seeking a full-arch fixed prothesis, may however have an existing denture.

This embodiment provides a technique for imaging an implant and correlating an imaging result with a denture and a related scan body. By imaging the intra-oral scan body and imaging the scan body in the extra-oral denture, the generated data can be matched to the scan body by software to position the two imaging scan bodies in the oral cavity and in the denture in relatively consistent positions. Therefore, the scan data of the extra-oral denture can be digitally "mapped" into the oral cavity for preparing the digital design of the fixed or implant-supported prosthesis, ensuring that the prosthesis can fit the patient's oral cavity well.

A specific process of the method is as follows.
(1) providing a patient having a denture who is desirous of a fixed prosthesis.
(2) optionally positioning the denture in the patient's oral cavity and performing an IOS (intra-oral scanning) scan of the denture and optionally the opposing teeth and optionally the bite.
(3) installing implants in the patient's maxilla or mandible.
(4) optionally affixing abutments to the implants.
(5) mounting primary scan bodies on the implants or optional abutments, where the scan bodies include PG (photogrammetry) fiducial markers.
(6) performing a PG scan of the primary scan bodies.
(7) removing the primary scan bodies and mounting healing collars to the abutments, where the healing collars include PG fiducial markers and are configured to provide a negative impression with a configuration to which a similar-shaped body can be mounted.
(8) optionally performing a PG scan of the healing collars.
(9) preparing an impression of the healing collars while mounted to the implants or optional abutments, where said impression is made within the intaglio surface of the denture or other device adapted to make impression, and where the impression produces a negative impression of a portion of the healing collars.
(10) mounting denture scan bodies into the negative impression of healing collar formed with the wash impression.
(11) performing a PG scan of the denture scan bodies while mounted within the impression as the impression is disposed on the intaglio surface of the denture or other device.
(12) performing an IOS scan of the denture and denture scan bodies while the denture scan bodies are mounted within the impression as the impression is disposed on the intaglio surface of the denture.
(13) correlating data obtained from the PG scan of the primary scan bodies with data obtained from the PG scan of the healing collars.
(14) correlating data obtained from the PG scan of the healing collars to data obtained from the PG scan of the denture scan bodies.
(15) correlating data obtained from the ISO scan of the denture scan bodies and denture with data obtained from the PG scan of the denture scan bodies.
(16) creating a digital image of a fixable prosthesis by using the correlated data.

In some embodiments, the denture may be an immediate denture, for example, before the implant is placed in the maxilla or mandible of the patient, the patient's oral cavity may also be imaged, the immediate denture is fabricated, then the patient's natural teeth are removed, and then the implant is placed in the maxilla or mandible of the patient.

In one or more embodiments, a denture imaging procedure is involved to prepare a fixed prosthesis (which may also be referred to as an implant-supported prosthesis). In one or more embodiments, the patient is a dental-free patient (wearing a denture such as a full-arch denture). In an optional step, the denture is placed in the patient's oral cavity, and the denture is subjected to pre-surgical scanning relative to other structures of the oral cavity (such as maxillary teeth). The pre-surgical scanning may be accomplished using a conventional scanning device, such as an optical three-dimensional scanning (IOS) device. The data acquired from the pre-surgical scanning is stored in a form of digital images for subsequent processing. In other embodiments, the patient may still have natural teeth (a dental patient), and the immediate denture reference is used. Those skilled in the art appreciate that such immediate dentures are usually fabricated according to upper and lower dental arch impressions and bite relationships, and then designed by denture processing. Such an immediate denture is a conventional prosthesis worn by a patient immediately after tooth extraction on the day of surgery. In the present disclosure, the immediate denture can play the same role as the existing denture of the patient, and its specific role will be described in detail below. For example, optical three-dimensional scanning (IOS) may be performed on an immediate denture tooth worn in a patient's oral cavity to obtain bite-related data.

Regardless of whether the patient has or doesn't have teeth, it is necessary to implant multiple implants in their oral cavity (i.e., in the maxilla or mandible) through conventional surgical procedures. For patients having teeth, it is necessary to remove the natural teeth first and then place the implant. The abutment may then be mounted (i.e., fixed or attached) to the implant using conventional techniques; in other embodiments, the abutment may not be mounted. As the abutment is used in most scenarios, the embodiments of the present disclosure will be described in combination with the abutment, but those skilled in the art can easily think of the implementation without using the abutment.

The primary scan body (also referred to as a primary reference member or a scannable fiducial carrier) is mounted on the abutment (for example, detachably mounted by fasteners such as screws), and photogrammetry scan or the like is performed on the primary scan body to determine the position and orientation of the implant and/or the abutment. In one or more embodiments, the primary scan body contains predetermined features for photogrammetric triangulation (e.g., at least two target points, such as dots, having a predetermined pattern and a known spacing). Images are acquired using conventional manners such as photogrammetry techniques, data related to abutment/implant position and orientation is generated, and is digitally stored in a form of digital images.

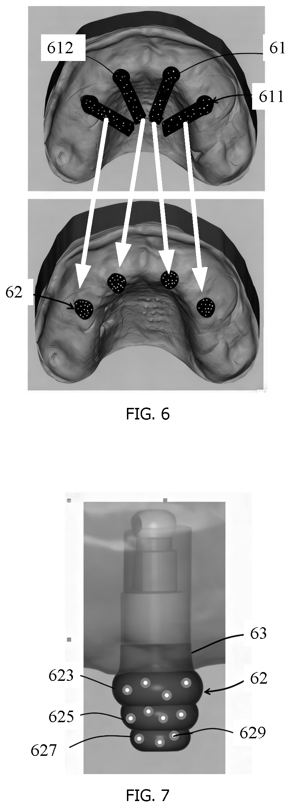

An exemplary embodiment can be described with reference to FIG. 6, which shows primary scan bodies 61 mounted to abutments (not shown), and the abutments are installed into implants (not shown) that surgically placed into the maxilla of a patient's mouth. Primary scan bodies 61 include a paddle portion 611 that, when installed, generally extends parallel to the bone surface into which the implants are installed. Scan bodies 61 include a plurality of dots 612, which have a predetermined pattern and known distances to facilitate photogrammetry triangulation.

In the next step of the process, scannable fiducial carriers are mounted to the abutments, and a physical impression of these fiducial carriers is obtained on the intaglio surface of a denture, or a stock or custom tray, or a device designed to capture the impression of the scannable fiducial carriers and the bite. In one or more embodiments, this includes the use of a wash impression whereby a thin layer of low-viscosity impression material is placed on the intaglio surface of the denture or other type of impression device mentioned above, and then the denture or other device is fitted into the mouth over the scannable fiducial carriers to record details relative to the position, shape dimensions and orientation of the fiducial carriers. As noted above, in the case of a dentulous patient, the immediate denture serves as the fiducial point providing tooth position, intaglio record and wash impression of the scan healing collars, and bite registration. However, on the day of surgery, the immediate denture will simply be used as the device for capturing a wash impression of the healing collars, and providing the laboratory with all the necessary records to make a prosthesis file to be screwed and fixed on the implants, once combined correlated to the other data, as will be described herein.

With regard to the scannable fiducial carriers, the fiducial carriers include a portion having a shape that is adapted to leave a negative impression in the wash impression such as that a similarly shaped body can be removably secured to the wash impression. In one or more embodiments, the scannable fiducial carriers include a scannable feature that allows the fiducial carriers to be scanned and a digital image thereof obtained while the fiducial carriers are mounted to the abutments. In another or more embodiments, the fiducial carriers include targets (e.g. dots) that facilitate photogrammetry imaging and data collection.

In one or more embodiments, the scannable fiducial carriers are the same as the primary scan bodies (i.e., mark carriers that are mounted to the abutments and scanned to determine the location and orientation of the implants/abutments as described above. In other embodiments, the scannable fiducial carriers are a separate and distinct set of scan bodies, which may also be referred to as secondary scan bodies or healing collars. In this regard, reference is made to FIG. 6, which shows and example healing collars 62 mounted to abutments (not shown), which are installed into implants (not shown), which are surgically placed into the maxilla of a patient's mouth. As shown in FIG. 7, these healing collars 62 are adapted to provide a negative impression in the wash impression such that a similarly shaped body can be secured into the wash impression. As shown, healing collars 62 include, for example, a stacked ring or donut structure where each successive ring (623, 625, 627) has a smaller diameter as the healing collar extends away from the abutment 63 to which it is mounted. Healing collars 62 also include a plurality of dots 629, which have a predetermined pattern and known distances to facilitate photogrammetry triangulation. In alternative embodiments (not shown), the healing collar can include a stacked ring structure where each successive ring has a larger diameter as the healer collar extends away from the abutment.

After the wash impression is prepared, which the skilled person appreciates includes removal of the denture impression material from the mouth, and after providing sufficient time to allow the impression material to set, denture scan bodies are inserted in the negative impressions of the healing collars within the wash impression. The denture scan bodies are then scanned to determine the location and orientation of the negative impression from the healing collars. In one or more embodiments, the step of scanning the denture scan bodies includes photogrammetry techniques whereby images are captured and the relative location and orientation of the negative impression of the healing collars is generated and stored electronically as a digital image.

In combination with the scan of the denture scan bodies, a scan can be made of the entire denture with, and optionally without, the denture scan bodies mounted to the negative impressions of the healing collars. In one or more embodiments, this scan of the entire denture is performed using IOS technology. This scan not only obtains data relative to the shape of the denture, but also data relative to the denture scan bodies. Therefore, those skilled in the art would appreciate that the techniques of the present disclosure obtain both IOS data and PG data relative to the denture scan bodies.

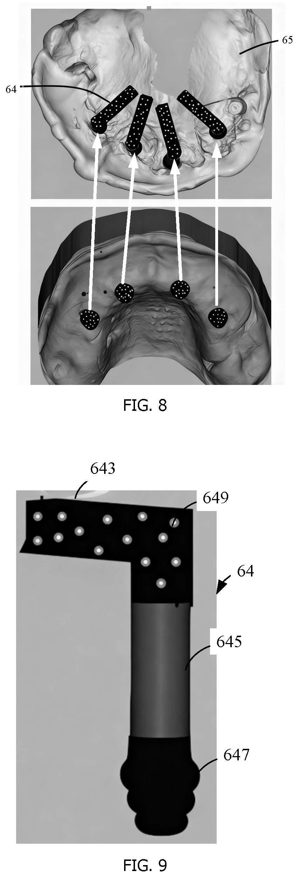

Further explanation can be made with reference to FIGS. 8 and 9, which show denture scan bodies 64 secured into the negative impressions (not shown) that are formed in wash impression 65. An example denture scan body 64 is shown in FIG. 9, where the denture scan body 64 includes a paddle portion 643, a body extension 645, and a mating portion 647. Paddle portion 643 includes a plurality of dots 649, which have a predetermined pattern and known distances to facilitate photogrammetry triangulation. Mating portion 647 is configured to removably mate with the negative impression formed in wash impression 65. As shown in FIG. 9, mating portion 647 has the same or substantially similar shape to healing collar 62 shown in FIG. 7.

Data collected from the foregoing scans (e.g. photogrammetry scans and/or IOS scans) is correlated to allow for the preparation of a digital image of a prothesis that is adapted to mate with the abutments mounted to the implants that are installed in the patient's mouth. For example, the prothesis may include apertures through which fastening devices can be installed and mated to the abutments.

The skilled person will appreciate that data relative to the position of the implants (and/or abutments) can be correlated with the position of the healing collars. This may include a first scan (e.g. PG scan) of the scan bodies mounted to the abutments and a second scan of healing collars mounted to the abutments. Data from each of these scans can be correlated to determine the position and orientation of the implants/abutments relative to the healing collars. In one or more embodiments, the healing collars can be registered (i.e. data relative to one or more characteristics of the collar such as its shape and/or positioning of targets can be stored within data storage in communication with a processing unit), which when correlated with the data from the scan can offer greater precision of the scan results and processing.

The data obtained from the scan of the denture scan bodies can then be correlated with the data relative to the position of the healing collars. Again, in one or more embodiments, the denture scan bodies can be registered (i.e. data relative to one or more characteristics of the scan bodies such as shape and/or positioning of targets can be stored within data storage in communication with a processing unit), which when correlated with the data from the scan can offer greater precision of the scan results and processing.

In one or more embodiments, the IOS data (i.e. digital image) obtained from the IOS scan of the denture and denture scan bodies is correlated with the PG data relative to the denture scan bodies.

Additionally, data obtained from the scan (e.g. pre-surgical) of the denture positioned within the mouth, including other references such as the opposing teeth, can be correlated to the post-surgical scan of the denture, which as noted above is correlated to other position of the implants/abutments.

From the foregoing, a digital design of a fixable (i.e. implant supportable) prothesis can be prepared, the prothesis fabricated, and ultimately affixed into the patient's mouth.

It shall be understood that the solutions described in the foregoing embodiments may be combined in a non-conflict basis, and are not listed one by one in the embodiments of the present disclosure.

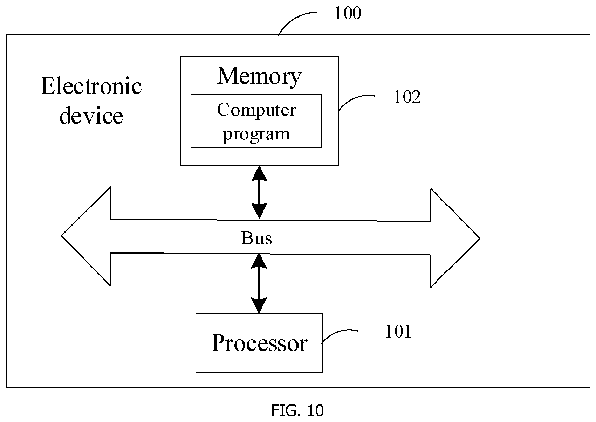

In addition, the present disclosure further provides an electronic device, as shown in FIG. 10, an electronic device 100 includes a processor 101, a memory 102, and a computer program stored in the memory 102 and executable by the processor 101, and when executing the computer program, the processor 101 may implement the method mentioned in any one of the foregoing embodiments.

Correspondingly, an embodiment of the present disclosure further provides a computer storage medium, where the storage medium stores a program, and when the program is executed by a processor, the method in any one of the foregoing embodiments is implemented.

Embodiments of the present disclosure may be in a form of a computer program product implemented on one or more storage media (including but not limited to a disk memory, a CD- ROM, an optical memory, and the like) that include program code. The computer usable storage medium includes permanent and non-permanent, removable and non-removable media, and information storage may be implemented by any method or technology. The information may be a computer-readable instruction, a data structure, a program module, or other data. Examples of a storage medium of a computer include, but are not limited to, a phase change memory (PRAM), a static random access memory (SRAM), a dynamic random access memory (DRAM), another type of random access memory (RAM), a read-only memory (ROM), an electrically erasable programmable read-only memory (EEPROM), a flash memory or another memory technology, a compact disc read-only memory (CD-ROM), a digital versatile disc (DVD) or another optical storage, a magnetic cassette tape, a magnetic tape magnetic disk storage or another magnetic storage device, or any other non-transmission medium, and may be configured to store information that can be accessed by a computing device.

For the apparatus embodiment, since it basically corresponds to the method embodiment, reference may be made to the partial description of the method embodiment for relevant parts. The apparatus embodiments described above are merely illustrative, where the units described as separate components may or may not be physically separate, and the components displayed as units may or may not be physical units, that is, may be located in one place, or may be distributed on multiple network units. Some or all of the modules may be selected according to actual needs to achieve the objectives of the solutions of the embodiments. Those skilled in the art can understand and implement without creative work.

It should be noted that, in this specification, relational terms such as first and second are only used to distinguish one entity or operation from another entity or operation, and do not necessarily require or imply that there is any such actual relationship or order between these entities or operations. The terms "include," "comprise," or any other variation thereof, are intended to cover a non-exclusive inclusion, such that a process, method, article, or device that comprises a list of elements does not include only those elements but may include other elements not expressly listed or inherent to such process, method, article, or device. An element proceeded by "comprises a ..." does not, without more constraints, preclude the existence of additional identical elements in the process, method, article, or device that comprises the element.

The method and device provided by the embodiments of the present disclosure are described in detail above, specific examples are used herein to illustrate the principle and implementation of the present disclosure, and the description of the above embodiments is only used to help understand the method and core idea of the present disclosure; meanwhile, for those of ordinary skill in the art, according to the idea of the present disclosure, there will be changes in the specific implementation and disclosure scope, and in summary, the content of the specification should not be construed as limiting the present disclosure.

## Claims

1. A data processing method, comprising:
acquiring first scan data by scanning an oral cavity of a patient in a first state, wherein the first state of the oral cavity of the patient indicates that at least one first scan body is mounted on an implant in the oral cavity of the patient, and the first scan data at least comprises scan data of the first scan body;
acquiring second scan data by scanning an impression in a preset state, wherein the impression is fabricated based on the oral cavity of the patient in the first state, the impression comprises a negative impression corresponding to at least a part of the first scan body, the impression in the preset state at least indicates that at least one second scan body is mounted in the impression, a partial shape of the second scan body is adapted to a shape of the negative impression, and the second scan body is detachably mounted in the negative impression, and the second scan data at least comprises scan data of the second scan body and scan data of the impression; and
acquiring target data based on the first scan data and the second scan data, wherein the target data is used to fabricate a dental prosthesis of the patient.

2. The method according to claim 1, wherein acquiring the target data based on the first scan data and the second scan data comprises:
determining first pose information of the implant based on the scan data of the first scan body;
determining second pose information of the implant based on the scan data of the second scan body;
determining morphological data of gingiva within the oral cavity of the patient based on the scan data of the impression; and
aligning the first scan data and the second scan data based on the first pose information and the second pose information to obtain the target data, wherein the target data comprises the morphological data of the gingiva and pose information of the implant.

3. The method according to claim 1, wherein the impression in the preset state indicates that the second scan body is mounted in the impression, and the impression is placed in a denture of the patient, and the second scan data further comprises morphological data of the denture.

4. The method according to claim 3, further comprising:
acquiring fourth scan data by scanning the oral cavity of the patient in a second state, wherein the second state of the oral cavity of the patient indicates that the denture of the patient is mounted in the oral cavity of the patient, and the fourth scan data at least comprises morphological data of the denture and intra-oral bite data of the denture; and
associating the fourth scan data with the second scan data based on the morphological data of the denture to obtain updated second scan data, and acquiring the target data based on the first scan data and the updated second scan data, wherein the updated second scan data further comprises the intra-oral bite data of the denture.

5. The method according to claim 3, wherein the denture is an interim denture temporarily fabricated for a patient, and the interim denture is fabricated by:
acquiring fifth scan data by scanning the oral cavity of the patient in a third state, wherein the third state of the oral cavity of the patient indicates that the oral cavity of the patient is in a pre-extraction state, and the fifth scan data at least comprises intra-oral bite data of the oral cavity of the patient in the pre-extraction state; and
generating an interim denture design model for the patient based on the fifth scan data, wherein the interim denture is fabricated based on the interim denture design model.

6. The method according to claim 1 or 2, wherein there are multiple second scan bodies, and each of the multiple second scan bodies is provided with a fiducial point, and acquiring the second scan data of the impression in the preset state comprises:
acquiring a first image frame set of the impression in the preset state, and acquiring respective fiducial point data of the multiple second scan bodies based on the first image frame set;
determining pose information and identification information of each of the multiple second scan bodies based on the fiducial point data of each of the multiple second scan bodies, and retrieving a standard model corresponding to each of the multiple second scan bodies from a standard library based on the identification information;
binding the pose information of the multiple second scan bodies with multiple standard models respectively based on a correspondence between the multiple standard models and the multiple second scan bodies to obtain intermediate standard data;
acquiring a second image frame set of the impression in the preset state, and obtaining morphological data of the multiple second scan bodies and morphological data of the impression based on the second image frame set; and
splicing the intermediate standard data and the morphological data of the impression based on a correspondence between each of the multiple standard models in the intermediate standard data and the actual morphological data of the multiple second scan bodies to obtain the second scan data.

7. The method according to claim 1, wherein a surface of the first scan body is provided with multiple fiducial markers, and the method further comprises:
acquiring sixth scan data of the oral cavity of the patient in a fourth state, wherein the fourth state of the oral cavity of the patient indicates that at least one third scan body is fixed in the implant in the oral cavity of the patient; wherein a surface of the third scan body is provided with multiple fiducial markers, and coverage of the multiple fiducial markers on the surface of the third scan body in the oral cavity of the patient is greater than coverage of the multiple fiducial markers on the surface of the first scan body in the oral cavity of the patient; and the sixth scan data at least comprises scan data of the third scan body; and
associating the sixth scan data with the first scan data based on the scan data of the third scan body to obtain updated first scan data, and acquiring the target data based on the updated first scan data and the second scan data.

8. The method according to claim 7, wherein the third scan body comprises a paddle portion and a body extension; wherein one end of the body extension is configured to be connected to the implant, and another end of the body extension is connected to the paddle portion, wherein the body extension and the paddle portion are of an L-shaped structure, and wherein a surface of at least one of the paddle portion or the body extension is provided with multiple fiducial markers.

9. The method according to claim 7, wherein the third scan body is detachably connected to a mating portion, the mating portion is adapted to a structure of the first scan body, and the second scan body is formed when the mating portion is connected to the first scan body.

10. The method according to claim 1 or 2, wherein after acquiring the target data, the method further comprises:
generating a design model of the dental prosthesis of the patient based on the target data.

11. The method according to claim 10, wherein after generating the design model of the dental prosthesis of the patient, the method further comprises:
sending the design model of the dental prosthesis to a fabricating device;
receiving, in real time, a user request to modify the design model of the dental prosthesis; and
outputting a revised design model of the dental prosthesis according to the user request and the target data.

12. The method according to claim 1, wherein the first scan body comprises a first end portion and a second end portion, the first end portion is configured to be connected to the implant, the second end portion is capable of forming the negative impression in an impression material, and an outer surface of the second end portion of the first scan body is provided with multiple fiducial markers.

13. The method according to claim 12, wherein the second end portion is configured as stacked rings, and a diameter of each of the stacked rings gradually decreases in a direction away from the first end portion.

14. The method according to claim 13, wherein axes of the stacked rings are collinear.

15. The method according to claim 1, wherein the second scan body comprises a paddle portion, a body extension and a mating portion; one end of the body extension is connected to the mating portion, and another end is connected to the paddle portion; a shape of the mating portion is adapted to a shape of the negative impression, and the mating portion is detachably mounted in the negative impression; and a surface of at least one of the paddle portion or the body extension is provided with multiple fiducial markers.

16. The method according to claim 15, wherein the body extension and the paddle portion are of an L-shaped structure.

17. The method according to claim 15, wherein the paddle portion, the body extension, and the mating portion are integrally formed; or
at least one of the paddle portion and the mating portion is detachably connected to the body extension.

18. The method according to claim 15, wherein the mating portion is configured as stacked rings, and a diameter of each of the stacked rings gradually decreases in a direction away from the body extension.

19. The method according to claim 1, wherein the impression is fabricated by:
fixing the first scan body on the implant, uniformly coating an impression material on an intaglio surface of an impression carrier, and placing the impression carrier into the oral cavity of the patient and covering the first scan body to fabricate the impression comprising the negative impression of the first scan body.

20. The method according to claim 19, wherein the impression carrier comprising any one of: an interim denture of the patient, an existing denture of the patient, a custom tray of the patient, and a stock tray.

21. An electronic device, comprising one or more processors, a memory, and a computer program stored in the memory and executable by the one or more processors, wherein the one or more processors, when executing the computer program, are caused to perform the method according to any one of claims 1-20.

22. A computer-readable storage medium, storing a computer program, wherein when the computer program is executed by one or more processors, the one or more processors are caused to perform the method according to any one of claims 1-20.
